# EUROPEAN PATENT SPECIFICATION

(11) **EP 4 400 598 B1**
(45) Date of publication and mention of the grant of the patent: **15.07.2026**
(21) Application number: 22863613.0
(22) Date of filing: 02.09.2022
(51) Int. Cl.: C12Q 1/6806, C12Q 1/6809, C40B 20/04

(54) **REAGENT AND METHOD FOR HIGH-THROUGHPUT SINGLE-CELL TARGETED SEQUENCING**
REAGENZ UND VERFAHREN ZUR GEZIELTEN HOCHDURCHSATZ-EINZELZELLENSEQUENZIERUNG
RÉACTIF ET PROCÉDÉ POUR LE SÉQUENÇAGE CIBLÉ HAUT DÉBIT DE CELLULES UNIQUES

(30) Priority: 02.09.2021 WO PCT/CN2021/116255
(43) Date of publication of application: 17.07.2024
(73) Proprietor: Singleron (Nanjing) Biotechnologies, Ltd., Nanjing, Jiangsu 211800 (CN)
(72) Inventor: ZHU, Wenqi, Nanjing, Jiangsu 211800 (CN); WEI, Xing, Nanjing, Jiangsu 211800 (CN)
(74) Representative: Witte, Weller und Partner Patentanwälte mbB Stuttgart
(86) International application number: PCT/CN2022/116674
(87) International publication number: WO 2023/030479

(56) References cited:
- WO-A1-2019/032762
- WO-A1-2020/157684
- WO-A1-2020/198532
- CN-A- 110 114 520
- CN-A- 110 819 706
- US-A1- 2018 346 969
- US-A1- 2019 360 044
- US-A1- 2021 024 977
- WANG QUAN; WANG ZHU; ZHANG ZHEN; LI CHEN; ZHANG MENGMENG; YE YINGJIANG; WANG SHAN; JIANG KEWEI: "Overview of the Technology of Single Cell Sequencing", CHINESE JOURNAL OF MEDICINAL GUIDE, vol. 22, no. 7, 15 July 2020 (2020-07-15), pages 433 - 439, XP055894854, ISSN: 1009-0959

## Description

### TECHNICAL FIELD

The present application relates generally to the field of molecular biology, and specifically provides a method for analyzing a cellular gene expression level and a target gene sequence at single-cell level.

### BACKGROUND

Single-cell transcriptome sequencing is the most popular technique in the field of biology in recent years. With its ultra-high resolution that enables accurate analysis of sample information, it has great application potentials in many sub-fields of biology. For example, it can be used to study the important effect of tumor heterogeneity on disease development and drug intervention and analyze immunoreceptor diversity, and can be translationally applied to vaccination, cancers, and autoimmune diseases. Cell phenotypic diversity in humans and other vertebrates can arise from complex gene rearrangement and alternative RNA splicing events^{[1,2]}. However, single-cell transcriptome sequencing, restricted by the current short-read mRNA library construction and sequencing techniques, shows marked selectivity for the 3' end or 5' end of transcripts, and cannot effectively capture the gene sequences distant from the 3' end or 5' end. It would be more difficult for single-cell transcriptome sequencing to detect complex gene rearrangement and RNA splicing.

For example, each newly-differentiated T or B lymphocyte in the immune system carries a different antigen receptor as the result of DNA rearrangements that alter the 450 nucleotides at the 5' end of their T- or B-cell antigen-receptor mRNA^{[3]}. In the case of B lymphocytes, they use additional DNA rearrangements to "isotype switch" between nine alternative constant region sequences comprising 1000-1500 nucleotides at the 3' end of the heavy chain mRNA^{[4]}, and use alternative mRNA splicing to change the nucleotides at the 3' end of IGH mRNA in order to secrete the encoded receptors as antibody^{[5]}. Similarly, complex gene rearrangements and alternative splicing events create pathological cell diversity amongst cancer cells. Hence, there is a critical need for methods that capture these sequence changes occurring throughout the length of mRNA molecules at single cell resolution, and integrate that information with gene-expression features.

Currently, existing technical protocols for single-cell targeted sequencing, such as the single-cell targeted sequencing products of 10X Genomics, are based on the methods of probe hybridization capture, and capture gene fragments of interest by probe hybridization on the basis of the original single cell 3' end or 5' end transcriptome library. While enrichment of the target genes is achieved, the problem of end preference is not solved, and genes distant from the 3' end or 5' end still cannot be captured. In the case of the targeted sequencing products of BD Biosciences, an enrichment panel is designed on the basis of different genes, and PCR amplification is performed on gene loci of interest by multiplex PCR, so as to acquire the expression information of multiple genes. However, this protocol, based on the strategy of mRNA capture at the 3' end, can only quantify the expression of the target genes and cannot obtain the variation information of the target genes. With respect to detection following gene rearrangement, for example, detection of immunoreceptor sequences, some methods and reagents are available fordetectingimmunoreceptors of single cells. For example, SMART-seq2-based capture of immunoreceptor sequences is available. However, this method relies on a flow cytometer for cell sorting, and suffers from a low detection throughput of typically 10 to 100 cells^{[6]}.

WO 2020/157684 A1 discloses a microwell-based single-cell transcriptome analysis method using bead-bound barcoded oligonucleotides comprising a cell barcode, a UMI and a polyT sequence to generate full-length cDNA by template switching. The cDNA may be circularized and amplified, for example by rolling circle amplification, to obtain transcriptome information, with optional mention of target gene enrichment.

WO 2019/032762 A1 discloses methods for circularization of barcoded cDNA followed by amplification using outward-facing primers in an inverse PCR configuration, enabling enrichment and sequencing of regions distant from the barcode, including immune receptor genes such as TCR and BCR.

The detection cost of single cells is high, and the BCR or transcript cannot be detected at the same time. Therefore, there is a need to develop a method for high-throughput detection of target genes of single cells (including, but are not limited to, immunoreceptor nucleic acid sequences) in order to achieve enrichment of targeted sequences of a great multitude of single cells.

### SUMMARY OF THE INVENTION

The present application provides a method for high-throughput single-cell targeted sequencing.

The present invention relates to a method for analyzing a cellular gene expression level and a target gene sequence at single-cell level, wherein the method comprises:
(a) loading a cell and a bead attached with multiple barcoded oligonucleotides together into a same microwell, wherein each of the multiple barcoded oligonucleotides comprises a cell barcode and a unique molecular identifier (UMI), wherein each of first barcoded oligonucleotidesof the multiple barcoded oligonucleotides comprises a polyT sequence capable of binding to a polyA tail of a first messenger ribonucleic acid (mRNA) target;
(b) following double-strand synthesis, allowing part of cDNA to undergo PCR amplification and cyclization to form circular double-stranded cDNA;
(c) using the circular double-stranded cDNA as a template to perform full-length enrichment of a target gene by inverse PCR using a strand-specific primer designed to enrich the target gene; and
(d) matching and analyzing transcriptome information and target gene information.

Preferably, the sequence of the multiple barcoded oligonucleotides comprises binding sites for two different sequencing primers, wherein the binding sites can be used as PCR primer binding sequences for amplifying the sequence of the cDNA.

Preferably, the sequence of the multiple barcoded oligonucleotides comprises binding sites for two different sequencing primers, wherein the binding sites can be used as sequencing primer binding sites for performing library sequencing.

Preferably, the cDNA cyclization is performed through an enzymatic reaction.

Preferably, the enzyme for the cDNA cyclization is a DNA ligase.

Preferably, the enzyme for the cDNA cyclization is a DNA polymerase.

Preferably, the information is obtained by sequencing.

Preferably, the matching and analyzing is performed by pairing following cell barcode identification.

Preferably, the target gene is a gene of a vertebrate.

### DESCRIPTION OF THE DRAWINGS

Fig. 1 is a diagram showing the principle of a high-throughput single-cell targeted sequencing experiment.
Fig. 2 is a quality inspection diagram of the full-length TCR cDNA in Example 1.
Fig. 3 is a quality inspection diagram of the full-length TCR enrichment library in Example 1.
Fig. 4 is a quality inspection diagram of the full-length BCR cDNA in Example 2.
Fig. 5 is a quality inspection diagram of the full-length BCR enrichment library in Example 2.
Fig. 6 is a quality inspection diagram of the Perturb-seq cDNA in Example 3.
Fig. 7 is a quality inspection diagram of the Perturb-seq transcriptome library in Example 3.
Fig. 8 is a product quality inspection diagram of the Perturb-seq target sequences in the three rounds of enrichment in Example 3.

### DETAILED DESCRIPTION OF THE PREFERRED EMBODIMENTS

Single-cell transcriptome sequencing integrating a single-cell targeted sequencing technique can analyze the cell type and the target gene sequence information, including cell mutation information, of a transcriptome at the same time, and is a powerful tool for studying the relationship between tumor cell development, targeted drug, and gene hot-spot mutations. Single-cell transcriptome, in conjunction with targeted mutation, enables accurate identification of the type of a mutated cell, providing guidance for clinical drug administration. At the same time, it enables dynamic monitoring of changes in mutation type and frequency during drug use. This technique involves allowing some of double-stranded cDNA to cyclize, and designing strand -specific primers to enrich a target gene distant from the 3' end by means of reverse PCR. On the basis of a unique single cell microfluidics system from Singleron, not only can a common single-cell transcriptome be detected, but also a target gene and an immunoreceptor sequence of interest can be obtained. The method disclosed herein not only enables acquisition of high-quality single-cell transcriptome data, but also enables acquisition, according to customer needs, of hot-spot mutation information of interest at a much lower sequencing depth than that for the transcriptome. The technique has the following characteristics: (1) high throughput: allowing for simultaneous detection of mutations in regions of interest of thousands of cells; (2) customized depth: allowing for designing corresponding enrichment primers according to different customer needs; and (3) high cost-effectiveness ratio: the experimental procedure being highly compatible with the single-cell transcriptome work flow. It is only necessary to customize primer sequences and construct a corresponding enrichment library in order to achieve capture of target regions.

Some aspects of the above embodiments will be disclosed in more detail in the following examples, which are not intended to limit the scope of the present disclosure in any way.

### Example 1: Full-length TCR sequence detection

**Experimental material:** Mouse spleen cells. Cell concentration: 2.5*10⁵/mL. Cell activity: 95%.

### Experimental procedure:

### 1. Single cell sorting, mRNA capture, reverse transcription, and PCR enrichment

(1) The mouse spleen cells were separately loaded into a Singleron microfluidics chip, achieving separation of individual cells according to the principle of "Poisson distribution".
(2) FL Barcoding Beads were loaded into the chip, ensuring that 90% of the wells were loaded with the beads. The Oligo sequence of the FL Barcoding Beads comprised an Illumina Read 1 sequencing primer sequence, a cell barcode, a molecular tag (UMI), and a poly-T nucleotide sequence. Capture of multiple mRNAs was achieved by means of the polyT.
(3) 100 µL of a cell lysis solution was loaded into the chip, and lysis was performed at room temperature for 15 min, achieving capture and labeling of mRNAs of individual cells.
(4) After lysis was complete, the beads in the chips were recovered using a 1.5 mL centrifuge tube. The beads were rinsed, and the supernatant was discarded. 200 µL of a reverse transcription reagent was added, and reaction was allowed at 42°C, 1300 rpm for 90 min, thereby achieving a reverse transcription process.
(5) After reverse transcription was completed, amplification was performed by means of the PCR handle sequence (adapting to the sequencing primers of the Illumina second-generation sequencing platform) at the 5' end of the FL Barcoding Beads and the TSO sequence added during the reverse transcription process so as to accomplish enrichment of the full-length transcriptome, thereby obtaining cDNA products.
(6) Some of the cDNA products obtained were used for the construction of the transcriptome library, and some were used for subsequent enrichment of full-length TCRs and BCRs.

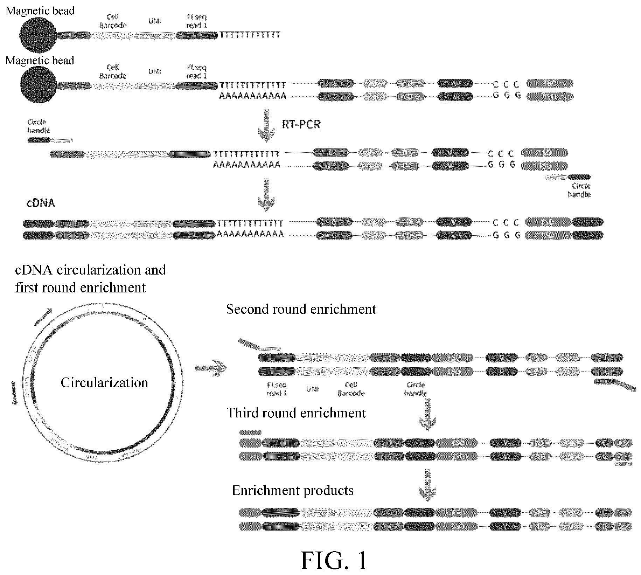

For the specific experimental operation in this section, reference can be made to the instructions of the GEXSCOPE single-cell RNAseq library construction kit (Singleron Biotechnologies). For the principle underlying the experiment in this section, reference can be made to Fig. 1.

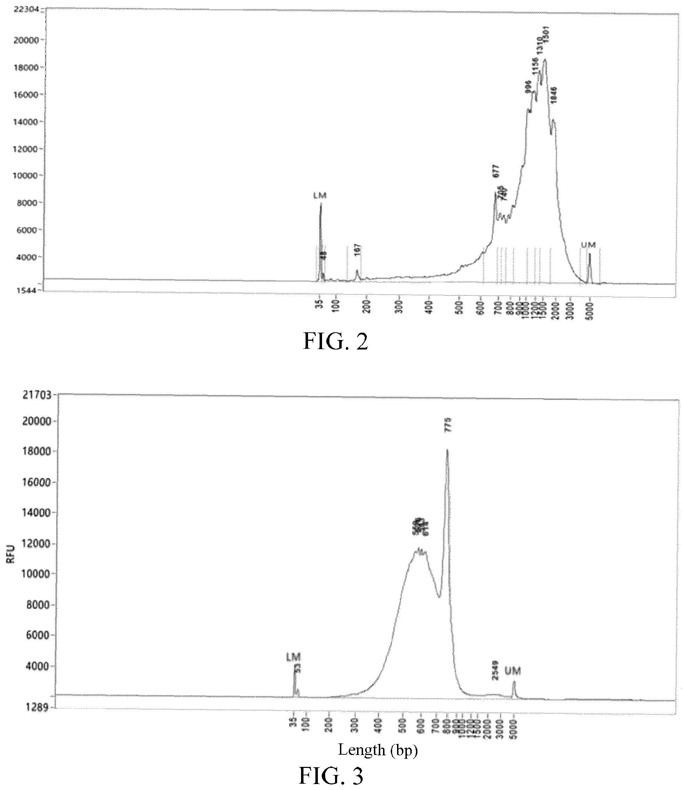

### 2. Full-length TCR enrichment and library construction (For the principle underlying the experiment in this section, reference can be made to Fig. 2.)

### 2.1 Cyclization

1) 250 ng of the cDNA products was loaded into a PCR tube. The PCR tube was placed on ice, and a cyclization mix was prepared according to the following table. The cyclization mix was mixed uniformly by vortexing, and centrifuged briefly.

| Component | 1 RXN (µL) |
|---|---|
| GA master mix | 10 |
| cDNA (250 ng) | Variable |
| Nuclease-free Water | Variable |
| Total | 20 |

2) The cyclization mix was gently mixed uniformly using a pipette. After brief centrifugation, the reaction tube was placed in a PCR instrument. The PCR instrument was set and reactions were run according to the following table, with the PCR hot cover being at 85°C:

| Step | Temperature | Time |
|---|---|---|
| 1 | 50°C | 1:10:00 |
| 2 | 75°C | 0:10:00 |
| 4 | 4°C | Hold |

3) After cyclization, the products, without being purified, were enzymatically cleaved by adding Cyclicase. The PCR tube was placed on ice, and an enzymatic cleavage system was prepared according to the table below.

| Component | Volume (µL) |
|---|---|
| Cyclization products in the previous step (products of Step 3.6.1.2) | 20 |
| Cyclicase | 0.5 |
| Nuclease-free Water | 4.5 |
| Total | 25 |

4) The enzymatic cleavage system was gently and sufficiently mixed uniformly using a pipette. After brief centrifugation, the reaction tube was placed in the PCR instrument. The PCR instrument was set and reactions were run according to the following table, with the PCR hot cover being closed:

| Step | Temperature | Time |
|---|---|---|
| 1 | 37°C | 30:00 |
| 2 | 4°C | Hold |

5) Product purification
(1) Magnetic beads (purification beads) were taken out from 4°C 30 min in advance, and were allowed to restore to room temperature.
   Note: The magnetic beads should be mixed uniformly before use.
(2) The liquid in the PCR tube was transiently centrifuged, and the volume was calculated. The magnetic beads were added in a volume of 1.3x of that of the products. After mixing uniformly by pipetting up and down, the mixture was incubated at room temperature for 5 min. The reaction was briefly centrifuged, and was allowed to stand on a magnetic stand for 5 min until the liquid became transparent and clear. The supernatant was carefully removed to a new PCR tube, and was held temporarily.
   Note: The magnetic beads are relatively viscous. A corresponding volume of the magnetic beads should be accurately measured, or else the length of the sorted fragments might be inconsistent with expectation.
(3) The PCR tube was consistently placed on the magnetic stand. 200 µL of newly prepared 80% ethanol was added to rinse the magnetic beads. The magnetic beads were incubated for 30 s at room temperature, and the supernatant was carefully removed.
(4) Step (3) was repeated, so that rinsing was performed two times in total.
(5) The PCR tube was removed and briefly centrifuged, and was then placed again on the magnetic stand. Excess alcohol was blotted off, and the PCR tube was allowed to air dry.
(6) The PCR tube was removed, and 20 µL of Nuclease-free Water was added. The magnetic beads were mixed uniformly by pipetting up and down, and were incubated for 5 min at room temperature. After brief centrifugation, the PCR tube was placed on the magnetic stand until the liquid became transparent and clear.
(7) The supernatant was drawn and transferred to a new EP tube, thereby obtaining the purified products.

### 2.2 First round enrichment of the full-length immunoreceptor (TCR)

1) 20 ng of the cyclization products were taken. A PCR tube was placed on ice, and a PCR mix for the first round enrichment was prepared according to the following table. The PCR mix was mixed uniformly by vortexing, and was centrifuged briefly.

| Component | 1 RXN (µL) |
|---|---|
| AL buffer | 10 |
| dNTP mix | 1.5 |
| Mus FL T1 primer | 1.5 |
| FL R1 primer | 1.5 |
| GEXSCOPE^{®} AL Enzyme | 1 |
| Nuclease-free Water | Variable |
| Cyclization products (20 ng) | Variable |
| Total | 50 |

2) The PCR mix was gently and sufficiently mixed uniformly using a pipette. After brief centrifugation, the reaction tube was placed on the PCR instrument. The PCR instrument was set and reactions were run according to the following table, with the PCR hot cover being at 105°C:

| Step | Temperature | Time |
|---|---|---|
| 1 | 98°C | 0:03:00 |
| | 98°C | 0:00:20 |
| 2 cycle=14 | 63°C | 0:00:45 |
| | 72°C | 0:02:00 |
| 3 | 72°C | 0:05:00 |
| 4 | 4°C | Hold |

3) Product purification

The products were purified using 0.6x magnetic beads, the specific steps being the same as described above.

### 2.3 Second round enrichment of the full-length immunoreceptor

1) 20 ng of the product of the first round enrichment was taken. A PCR tube was placed on ice, and a PCR mix for the first round enrichment was prepared according to the following table. The PCR mix was mixed uniformly by vortexing, and was centrifuged briefly.

| Component | 1 RXN (µL) |
|---|---|
| AL buffer | 10 |
| dNTP mix | 1.5 |
| Mus FL T2 primer | 1.5 |
| FL R2 primer | 1.5 |
| GEXSCOPE^{®} AL Enzyme | 1 |
| Nuclease-free Water | Variable |
| Products of the first round enrichment (20 ng) | Variable |
| Total | 50 |

2) The PCR mix was gently and sufficiently mixed uniformly using a pipette. After brief centrifugation, the reaction tube was placed on the PCR instrument. The PCR instrument was set and reactions were run according to the following table, with the PCR hot cover being at 105°C:

| Step | Temperature | Time |
|---|---|---|
| 1 | 98°C | 0:03:00 |
| | 98°C | 0:00:20 |
| 2 cycle=10 | 65°C | 0:00:45 |
| | 72°C | 0:02:00 |
| 3 | 72°C | 0:05:00 |
| 4 | 4°C | Hold |

3) Product sorting
(1) Purification magnetic beads were taken out 30 min in advance, and were allowed to restore to room temperature for later use. The PCR tube was transiently centrifuged, and the volume of the PCR products was measured. 25 µL of magnetic beads (0.5x of the volume of the products) was added. The mixture was sufficiently mixed uniformly by vortex oscillation or by pipetting 10 times with a pipette, and was incubated at room temperature for 5 min.
(2) The PCR tube was centrifuged briefly and then placed on the magnetic stand to allow the magnetic beads to separate from the liquid. After the solution become clear (about 5 min), the supernatant was carefully transferred to a new sterile PCR tube.
(3) 7.5 µL (0.15x of the volume of the products) of the magnetic beads was drawn to the supernatant collected in step 2). The mixture was sufficiently mixed uniformly by vortex oscillation or by pipetting 10 times with a pipette, and was incubated at room temperature for 5 min.
(4) The PCR tube was centrifuged briefly and then placed on the magnetic stand to allow the magnetic beads to separate from the liquid. After the solution become clear (about 5 min), the supernatant was carefully removed.
(5) The PCR tube was consistently placed on the magnetic stand. 200 µL of freshly prepared 80% ethanol was added to rinse the magnetic beads. The magnetic beads were incubated for 30 s at room temperature, and the supernatant was carefully removed.
(6) Step (5) was repeated, so that rinsing was performed two times in total.
(5) The PCR tube was removed from the magnetic stand and briefly centrifuged, and was then placed again on the magnetic stand. Excess alcohol was blotted off, and the cover was opened to expose the magnetic beads in air for 2 min for drying.
(8) The PCR tube was removed from the magnetic stand, and 20 µL of Nuclease-free Water was added for elution. Vortex oscillation was performed to sufficiently mix the magnetic beads and the liquid uniformly, and the mixture was incubated at room temperature for 5 min.
(9) The PCR tube was centrifuged briefly and placed on the magnetic stand to allow the magnetic beads to separate from the liquid. After the solution become clear (about 5 min), 18 µL of the supernatant was carefully drawn to a new sterile PCR tube.

### 4) Quality inspection of the amplified and purified products

1 µL of the sample was taken for Qubit concentration detection.

### 3.4 Third round enrichment of the full-length immunoreceptor (TCR)

(1) 20 ng of the products of Step 3.3 was taken. A PCR tube was placed on ice, and a PCR mix for the third round enrichment was prepared according to the following table. The PCR mix was mixed uniformly by vortexing, and was centrifuged briefly.

| Component | Volume (µL) |
|---|---|
| AL buffer | 10 |
| dNTP mix | 1.5 |
| FL T3 primer | 1.5 |
| FL R3 primer | 1.5 |
| Amplification Enzyme | 1 |
| Nuclease-free Water | Variable |
| Products of Step 3.3 | Variable |
| Total | 50 |

2) The reaction tube was placed on the PCR instrument. The PCR instrument was set and reactions were run according to the following table, with the PCR hot cover being at 105°C:

| Step | Temperature | Time |
|---|---|---|
| 1 | 98°C | 0:03:00 |
| 2 cycle=6 | 98°C | 0:00:20 |
| | 64°C | 0:00:30 |
| | 72°C | 0:01:00 |
| 3 | 72°C | 0:05:00 |
| 4 | 4°C | Hold |

The products were purified using 0.6x magnetic beads, the specific steps being the same as described above.

### 3.5 Construction of a full-length immunoreceptor enrichment library

### 3.5.1 Fragmentation

(1) A PCR procedure was set up in advance according to the table below, with the reaction volume being 35 µL, and the PCR hot cover being maintained at 75°C.

| Step | Temperature | Time |
|---|---|---|
| 1 | 4°C | Hold |
| 2 | 37°C | 0:05:00 |
| 3 | 65°C | 0:30:00 |
| 4 | 4°C | Hold |

(2) FR Buffer V2 and 1xTE were thawed at room temperature, ensuring complete melting. Then, they were mixed uniformly by vortexing for 10s, and were centrifuged briefly and placed on ice for later use. Before use, FR Mix V2 was vortex oscillated for 10 s, and was placed on ice for later use.
(3) A sterile PCR tube was placed on ice, and the following reaction system was prepared:

| Component | Volume (µL) |
|---|---|
| FR Buffer V2 | 7 |
| FR Mix V2 | 2 |
| Enrichment products | Variable (50ng) |
| 1xTE | Variable |
| Total | 35 |

(4) The reaction system was sufficiently mixed uniformly by gently pipetting with a pipette. The PCR tube was transiently centrifuged and then placed on the PCR instrument. The first step of the PCR procedure was skipped.
(5) After the reaction was completed, the next step of linker ligation was performed immediately.

### 3.5.2 Linker ligation

(1) A PCR procedure was set up in advance according to the table below, with the reaction system being 70 µL, and the PCR hot cover heating function being disabled.

| Step | Temperature | Time |
|---|---|---|
| 1 | 4°C | Hold |
| 2 | 20°C | 0:15:00 |
| 3 | 4°C | Hold |

(2) AFI was thawed at room temperature in advance, and was mixed uniformly by vortexing for 10 s and then placed on ice for later use. Before use, LA Mix and LA IB were mixed uniformly by vortexing for 10 s, and then centrifuged briefly and placed on ice for later use. LA Mix is relatively viscous. When drawing LA Mix, care should be taken to measure an accurate volume (Separate addition of AFI is recommended in the case of a multi-reaction system).
(3) The PCR tube of the previous step was placed on ice, and the following reaction system was prepared:

| Component | Volume (µL) |
|---|---|
| Fragmentation products | 35 |
| LA Mix | 30 |
| LA IB | 1 |
| AFI | FIG. 2.5 |
| Total | 68.5 |

### 3.5.3 Product purification after linker ligation

(1) The linker ligation products were purified with a 0.5x purification coefficient using the same method as described above, except that 17 µL of O.1xTE (obtained by diluting 1xTE 1:9 using the Nuclease-free Water) was added during product resuspension, and the magnetic beads were mixed uniformly by vortex oscillation for 15 s, and were incubated at room temperature for 5 min.
(2) The PCR tube containing the purification magnetic beads was briefly centrifuged and was then placed on the magnetic stand to allow the magnetic beads to separate from the liquid. After the solution became clear (about 5 min), 15 µL of the supernatant was carefully drawn to a new sterile PCR tube for PCR enrichment.

### 3.5.4 PCR enrichment

(1) The PCR tube was placed on ice, and the following reaction system was prepared. The reaction system was mixed uniformly by gently pipetting 5 times with a pipette (45 µL range), and was centrifuged briefly. The reaction tube was placed on the PCR instrument.

| Component | Volume (µL) |
|---|---|
| Products obtained after linker ligation and purification | 15 |
| TE Adapter Mix | 10 |
| LP Buffer V2 | 25 |
| Total | 50 |

(2) The PCR procedure was set up in advance according to the table below. The PCR hot cover was maintained at 105°C, and the reaction volume was 50 µL.

| Step | Temperature | Time |
|---|---|---|
| 1 | 98°C | 0:00:30 |
| 2 (cycle=9) | 98°C | 0:00:10 |
| | 65°C | 0:01:15 |
| 3 | 65°C | 0:05:00 |
| 4 | 4°C | Hold |

### 3.5.5 Product sorting

The products were sorted with 0.5x and 0.15x sorting coefficients using the same method as described above. The information of the sequence and expression amount of VDJ(TCR) was obtained by library sequencing.

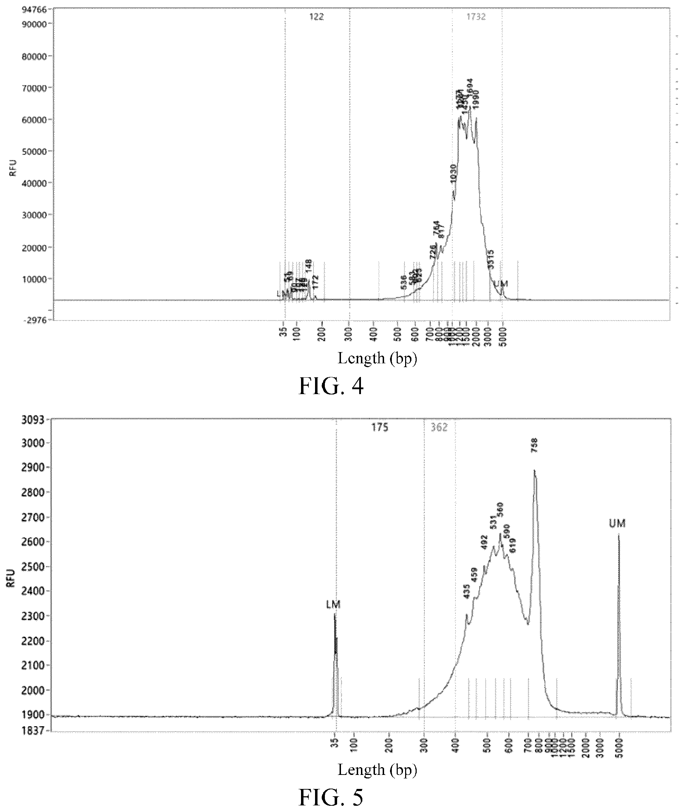

The experimental results are as shown in Fig. 3, which is a quality inspection diagram of the full-length TCR cDNA, and in Fig. 4, which is a quality inspection diagram of the full-length TCR enrichment library. The following Table 1 is a list of the top 10 clonetypes for the full-length TCR enrichment, and Table 2 is a list of important indexes for the full-length TCR enrichment.

**Table 1**

| **TOP10 Clonetypes** | | | |
|---|---|---|---|
| Clonotype ID | CDR3_aa | Frequency | Proportion |
| 1 | TRB:CASSQEGLVYEQYF | 258 | 4.42% |
| 2 | TRA:CAMREEMDTGRRALTF | 72 | 1.23% |
| | TRB:CASSQEGLVYEQYF | | |
| 3 | TRA:CAREGYQRVTF | 39 | 0.67% |
| 4 | TRB:CASSPWGQTSTDTQYF | 33 | 0.57% |
| 5 | TRA:CAREGYQKVTF | 28 | 0.48% |
| | TRB:CASSETGASYYGYTF | | |
| 6 | TRA:CAREGYQKVTF | 27 | 0.46% |
| | TRD:CASSQEGLVYEQYF | | |
| 7 | TRB:CASSETGASYYGYTF | 21 | 0.36% |
| 8 | TRB:CASSPLAGVODTQYF | 19 | 0.33% |
| 9 | TRA:CAVVNTGFQKLVF | 16 | 0.27% |
| | TRB:CASTQSTDTIQYF | | |
| 10 | TRA:CAVVNTGFQKLVF | 14 | 0.24% |
| | TRB:CASSQEGLVYEQYF | | |

**Table 2**

| Analysis Indexes | **Mu_0714pz_3_3Nlib** |
|---|---|
| **Reads Mapped to Any V(D)J Gene** | **21,302,676(78.8%)** |
| **Cells With Productive V-J Spanning (TRA, TRB) Pair** | **2,526(77.06%)** |
| **matched Cells With Productive V-J Spanning(TRA, TRB) Pair** | **1,949(83.4%)** |
| **Reads Mapped to TRA** | **10,732,440(39.7%)** |
| **Reads Mapped to TRB** | **10,516,169(38.9%)** |
| **Estimated Number of Cells** | **3278** |
| **Cells match with scRNA-seq analysis** | **2337** |
| **Median used TRA UMIs per Cell** | **7** |
| **Median used TRB UMIs per Cell** | **13** |
| **matched Cells With Productive V-J Spanning Pair** | **1,949(83.4%)** |
| **matched Cells With TRA Contig** | **2,010(86.01%)** |
| **matched Cells With CDR3-annotated TRA Contig** | **2,010(86.01%)** |
| **matched Cells With V-J Spanning TRA Contig** | **2,010(86.01%)** |
| **matched Cells With Productive TRA Contig** | **2,010(86.01%)** |
| **matched Cells With TRB Contig** | **2,276(97.39%)** |
| **matched Cells With CDR3-annotated TRB Contig** | **2,276(97.39%)** |
| **matched Cells With V-J Spanning TRB Contig** | **2,276(97.39%)** |
| **matched Cells With Productive TRB Contig** | **2,276(97.39%)** |

### Example 2 Full-length BCR sequence detection

**Experimental material:** Mouse PBMC cells. Cell concentration: 2.3*10⁵/mL. Cell activity: 92%.

**Experimental methods:** Except that the BCR enrichment procedure was different from the TCR enrichment procedure described above, other operations of the experimental methods were the same as those in the full-length TCR sequence detection, including single-cell sorting, mRNA capture, reverse transcription and PCR enrichment, cDNA cyclization, and enrichment product library construction. The specific steps of the enrichment procedure for the full-length BCR sequence detection are as follows:
**1. First round enrichment of the full-length immunoreceptor (BCR)**
1) 20 ng of the cyclization products were taken. A PCR tube was placed on ice, and a PCR mix for the first round enrichment was prepared according to the following table. The PCR mix was mixed uniformly by vortexing, and was centrifuged briefly.

| Component | 1 RXN (µL) |
|---|---|
| MP master mix | 25 |
| Mus FL B1 primer | 2.85 |
| FL R1 primer | 1.5 |
| Nuclease-free Water | 10.65 |
| Cyclization products (20 ng) | 10 |
| Total | 50 |

2) The PCR mix was gently and sufficiently mixed uniformly using a pipette. After brief centrifugation, the reaction tube was placed on the PCR instrument. The PCR instrument was set and reactions were run according to the following table, with the PCR hot cover being at 105°C:

| Step | Temperature | Time |
|---|---|---|
| 1 | 95°C | 0:15:00 |
| | 94°C | 0:00:30 |
| 2 cycle=14 | 60°C | 0:01:30 |
| | 72°C | 0:01:30 |
| 3 | 72°C | 0:10:00 |
| 4 | 4°C | Hold |

3) Product purification The product was purified with a 0.6x purification coefficient using the same purification method as described above.
**2. Second round enrichment of the full-length immunoreceptor (BCR)**
1) 20 ng of the products of the first round enrichment was taken. A PCR tube was placed on ice, and a PCR mix for the first round enrichment was prepared according to the following table. The PCR mix was mixed uniformly by vortexing, and was centrifuged briefly.

| Component | 1 RXN (µL) |
|---|---|
| MP master mix | 25 |
| Mus FL B2 primer | 2.72 |
| FL R2 primer | 1.5 |
| Nuclease-free Water | 2.78 |
| Products of Step 3.6.3.1 | 18 |
| Total | 50 |

2) The PCR mix was gently and sufficiently mixed uniformly using a pipette. After brief centrifugation, the reaction tube was placed on the PCR instrument. The PCR instrument was set and reactions were run according to the following table, with the PCR hot cover being at 105°C:

| Step | Temperature | Time |
|---|---|---|
| 1 | 95°C | 0:15:00 |
| | 94°C | 0:00:30 |
| 2 cycle=8 | 60°C | 0:01:30 |
| | 72°C | 0:01:30 |
| 3 | 72°C | 0:10:00 |
| 4 | 4°C | Hold |

3) Product purification The product was purified with a 0.6x purification coefficient using the same purification method as described above.
**3. Third round enrichment of the full-length immunoreceptor (BCR)**
1) 20 ng of the products of Step 4.3 was taken. A PCR tube was placed on ice, and a PCR mix for the third round enrichment was prepared according to the following table. The PCR mix was mixed uniformly by vortexing, and was centrifuged briefly.

| Component | Volume (µL) |
|---|---|
| AL buffer | 10 |
| dNTP mix | 1.5 |
| FL T3 primer | 1.5 |
| FL R3 primer | 1.5 |
| Amplification Enzyme | 1 |
| Nuclease-free Water | Variable |
| Products of Step 4.3 | Variable |
| Total | 50 |

2) The reaction tube was placed on the PCR instrument. The PCR instrument was set and reactions were run according to the following table, with the PCR hot cover being at 105°C:

| Step | Temperature | Time |
|---|---|---|
| 1 | 98°C | 0:03:00 |
| | 98°C | 0:00:20 |
| 2 cycle=6 | 64°C | 0:00:30 |
| | 72°C | 0:01:00 |
| 3 | 72°C | 0:05:00 |
| 4 | 4°C | Hold |

### 3. Product Purification

The product was purified with a 0.6x purification coefficient using the same purification method as described above.

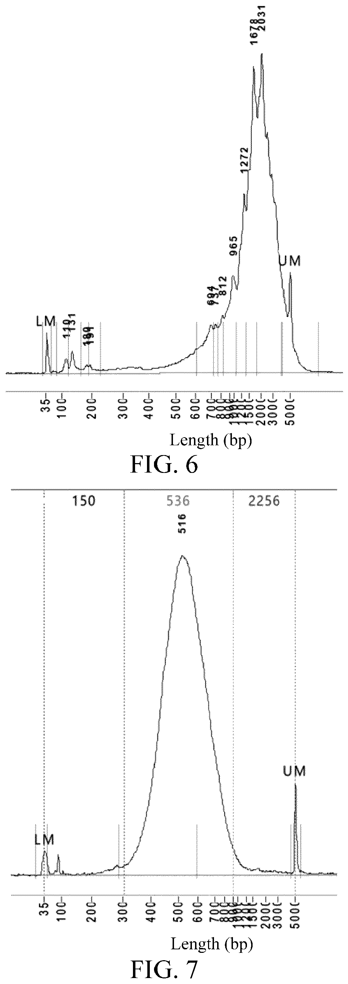

The experimental results are as shown in Fig. 5, which is a quality inspection diagram of the full-length BCR cDNA, and in Fig. 6, which is a quality inspection diagram of the full-length BCR enrichment library. The following Table 3 is a list of the top 10 clonetypes for the full-length BCR enrichment, and Table 4 is a list of important indexes for the full-length BCR enrichment.

**Table 3**

| **TOP 10 Clonetypes** | | | |
|---|---|---|---|
| Clonotype ID | Cdr3s_aa | Frequency | Proportion |
| clonotype4251 | IGH:CMRYSNYWYFDVW | 18 | 0.35% |
| | IGK:CLQHGESPYTF | | |
| clonotype106 | IGH:CAGVLYYYGSSYWYFDVW | 12 | 0.23% |
| | IGK:CQQGNTLPWTF | | |
| clonotype4249 | IGH:CMRYSNYWYFDVW | 10 | 0.19% |
| | IGK:CLQHGESPFTF | | |
| clonotype4234 | IGH:CMRYGSSYWYFDVW | 7 | 0.14% |
| | IGK:CLQHGESPFTF | | |
| clonotype4970 | IGL:CGVGDTIKEQFVYVF | 6 | 0.12% |
| clonotype4229 | IGH:CMRYGNYWYFDVW | 4 | 0.08% |
| | IGK:CLQHGESPFTF | | |
| clonotype4880 | IGK:CLQSDNMPLTF | 4 | 0.08% |
| clonotype4949 | IGK:CQQYSSYPLTF | 4 | 0.08% |
| clonotype4908 | IGK:CQQGNTLLPWTF | 4 | 0.08% |
| clonotype4226 | IGH:CMRYGGYWYFDVW | 4 | 0.08% |
| | IGK:CLQHGESPYTF | | |

**Table 4**

| Analysis Indexes | **M_0427PBMC1_N_B3Nlib** |
|---|---|
| **Estimated_Number_of_Cells** | **15,099** |
| **Cells_Match_with_ScRNA-seq_Analysis** | **6,001** |
| **Reads_Mapped_To_Any_V(D)J_Gene** | **79.09%** |
| **Cells_With_Productive_V-J_Spanning_Pair** | **97.67%** |
| **Cells_With_Productive_V-J_Spanning_(IGH,_IGL)_Pair** | **10.61%** |
| **Cells_With_Productive_V-J_Spanning_(IGH,_IGK)_Pair** | **90.13%** |
| **Cells_With_IGH_Contig** | **99.55%** |
| **Cells_With_Productive_IGH_Contig** | **98.20%** |
| **Cells_With_IGL_Contig** | **43.33%** |
| **Cells_With_Productive_IGL_Contig** | **10.86%** |
| **Cells_With_IGK_Contig** | **99.48%** |
| **Cells_With_Productive_IGK_Contig** | **91.78%** |

### Example 3: Perturb-seq target sequence enrichment

**Experimental material:** Mouse cell line. Cell concentration: 5.28*10⁶/mL. Cell activity: 95%.

### Experimental methods:

- The present invention could also be used for targeted capture of sgRNA. The difference between the capture magnetic beads used and theFL Barcoding beads in the above examples was that the magnetic beads were added, on the basis of the polyT probe, with a targeted probe sequence according to a certain proportion so as to achieve capture of mRNA and target sequences at the same time.
- The method of perturb-seq target sequence enrichment also involved performing cDNA template cyclization followed by performing enrichment. The primers for PCR enrichment bound to the constant region of sgRNA.
- Single-cell sorting, mRNA capture, reverse transcription and PCR enrichment, and cDNA cyclization were the same as those in the full-length TCR sequence detection described above. The specific steps of perturb-seq target sequence enrichment are as follows:

### 1. First round of perturb-seq target sequence enrichment

1) 10 µL of the cyclization products were taken. A PCR tube was placed on ice, and a PCR mix for the first round enrichment was prepared according to the following table. The PCR mix was mixed uniformly by vortexing, and was centrifuged briefly.

| Component | Volume (µL) |
|---|---|
| AL buffer | 10 |
| dNTP mix | 1.5 |
| CRISPR_R1_primer | 1.5 |
| FL R1 primer | 1.5 |
| Amplification Enzyme | 1 |
| Nuclease-free Water | FIG. 24.5 |
| Cyclization products | 10 |
| Total | 50 |

2) The reaction tube was placed on the PCR instrument. The PCR instrument was set and reactions were run according to the following table, with the PCR hot cover being at 105°C:

| Step | Temperature | Time |
|---|---|---|
| 1 | 98°C | 0:03:00 |
| | 98°C | 0:00:20 |
| 2 cycle=14 | 63°C | 0:00:45 |
| | 72°C | 0:02:00 |
| 3 | 72°C | 0:05:00 |
| 4 | 4°C | Hold |

3) Product purification

The product was purified with a 1x purification coefficient using the same purification method as described above.

### 2. Second round of perturb-seq target sequence enrichment

1) 18 µL of the cyclization products of Step 3.4.2.2 were taken. A PCR tube was placed on ice, and a PCR mix for the second round enrichment was prepared according to the following table. The PCR mix was mixed uniformly by vortexing, and was centrifuged briefly.

| Component | Volume (µL) |
|---|---|
| AL buffer | 10 |
| dNTP mix | 1.5 |
| CRISPR_R2_primer | 1.5 |
| FL R2 primer | 1.5 |
| Amplification Enzyme | 1 |
| Nuclease-free Water | FIG. 16.5 |
| First round products | 18 |
| Total | 50 |

2) The reaction tube was placed on the PCR instrument. The PCR instrument was set and reactions were run according to the following table, with the PCR hot cover being at 105°C:

| Step | Temperature | Time |
|---|---|---|
| 1 | 98°C | 0:03:00 |
| | 98°C | 0:00:20 |
| 2 cycle=12 | 65°C | 0:00:45 |
| | 72°C | 0:02:00 |
| 3 | 72°C | 0:05:00 |
| 4 | 4°C | Hold |

3) Product purification

The product was purified with a 1x purification coefficient using the same purification method as described above.

### 3. Third round of perturb-seq target sequence enrichment

1) 20 ng of the products of Step 3.4.2.2 were taken. A PCR tube was placed on ice, and a PCRmix for the third round enrichment was prepared according to the following table. The PCR mix was mixed uniformly by vortexing, and was centrifuged briefly.

| Component | Volume (µL) |
|---|---|
| AL buffer | 10 |
| dNTP mix | 1.5 |
| N701 primer | 1.5 |
| FL R3 primer | 1.5 |
| Amplification Enzyme | 1 |
| Nuclease-free Water | Variable |
| Products of the second round enrichment | Variable |
| Total | 50 |

2) The reaction tube was placed on the PCR instrument. The PCR instrument was set and reactions were run according to the following table, with the PCR hot cover being at 105°C:

| Step | Temperature | Time |
|---|---|---|
| 1 | 98°C | 0:03:00 |
| | 98°C | 0:00:20 |
| 2 cycle=10 | 64°C | 0:00:30 |
| | 72°C | 0:01:00 |
| 3 | 72°C | 0:05:00 |
| 4 | 4°C | Hold |

3) Product purification

The product was purified with a 0.8x purification coefficient using the same purification method as described above.

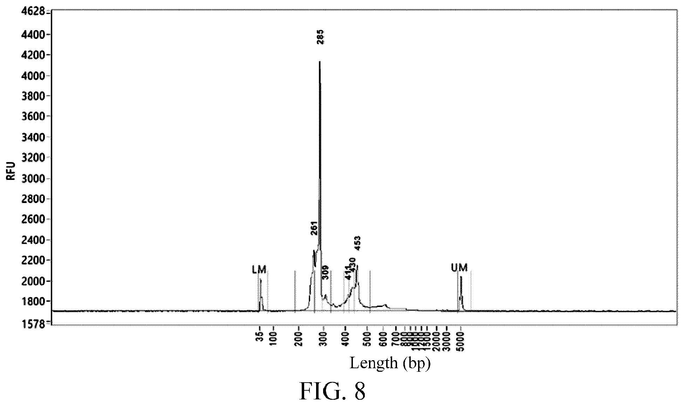

The experimental results are as shown in Fig. 7, which is a quality inspection diagram of Perturb-seq cDNA; in Fig. 8, which is a quality inspection diagram of Perturb-seq transcriptome library; and in Fig. 9, which is a quality inspection diagram of the Perturb-seq target sequences in the three rounds of enrichment.

### References:

[1] V(D)J Recombination and the Evolution of the Adaptive Immune System[J]. PLoS Biology, 2003, 1(1):e16-.
[2] Eric T, Wang, Rickard, et al. Alternative isoform regulation in human tissue transcriptomes.[J]. Nature, 2008.
[3] Bassing C H , Swat W , Alt F W . The Mechanism and Regulation of Chromosomal V(D)J Recombination[J]. Cell, 2002, 109(2):S45-S55.
[4] Chaudhuri J , Alt F W . Class-switch recombination: interplay of transcription, DNA deamination and DNA repair[J]. Nature Reviews Immunology.
[5] Frederick, W, Alt, et al. Synthesis of secreted and membrane-bound immunoglobulin mu heavy chains is directed by mRNAs that differ at their 3' ends[J]. Cell, 1980, 20(2):293-301.
[6] Picelli,S.et al.Smart-seq2 for sensitive full-length transcriptome profiling in single cells.Nat.Methods 10, 1096-1098,(2013).

## Claims

1. A method for analyzing a cellular gene expression level and a target gene sequence at single-cell level, the method comprising:
(a) loading a cell and a bead attached with multiple barcoded oligonucleotides together into a same microwell, wherein each of the multiple barcoded oligonucleotides comprises a cell barcode and a unique molecular identifier (UMI), wherein each of first barcoded oligonucleotides of the multiple barcoded oligonucleotides comprises a polyT sequence capable of binding to a polyA tail of a first messenger ribonucleic acid (mRNA) target;
(b) following double-strand synthesis, allowing part of cDNA to undergo PCR amplification and cyclization to form circular double-stranded cDNA;
(c) using the circular double-stranded cDNA as a template to perform full-length enrichment of a target gene by inverse PCR using a strand-specific primer designed to enrich the target gene; and
(d) matching and analyzing transcriptome information and target gene information.

2. The method according to claim 1, wherein the sequence of the multiple barcoded oligonucleotides comprises binding sites for two different sequencing primers, wherein the binding sites can be used as PCR primer binding sequences for amplifying the sequence of the cDNA.

3. The method according to claim 1, wherein the sequence of the multiple barcoded oligonucleotides comprises binding sites for two different sequencing primers, wherein the binding sites can be used as sequencing primer binding sites for performing library sequencing.

4. The method according to claim 1, wherein the cDNA cyclization is performed through an enzymatic reaction.

5. The method according to claim 4, wherein the enzyme for the cDNA cyclization is a DNA ligase.

6. The method according to claim 4, wherein the enzyme for the cDNA cyclization is a DNA polymerase.

7. The method according to claim 1, wherein the information is obtained by sequencing.

8. The method according to claim 1, wherein the matching and analyzing is performed by pairing following cell barcode identification.

9. The method according to claim 1, wherein the target gene is a gene of a vertebrate.

10. The method of claim 1, wherein the strand-specific primer is designed to enrich the target gene distant from the 3' end.

11. The method according to claim 1, wherein the enrichment comprises at least three rounds of enrichment.

12. The method according to claim 1, wherein the target gene encodes T cell receptor (TCR) or B cell receptor (BCR).

## Patentansprüche

1. Verfahren zur Analyse eines zellulären Genexpressionsniveaus und einer Zielgensequenz auf Einzelzellebene, wobei das Verfahren aufweist:
(a) gemeinsames Einbringen einer Zelle und eines Beads, an das mehrere barcodierte Oligonukleotide gebunden sind, in dieselbe Mikrowell, wobei jedes der mehreren barcodierten Oligonukleotide einen Zellbarcode und einen eindeutigen molekularen Identifikator (UMI) aufweist, wobei jedes der ersten barcodierten Oligonukleotide der mehreren barcodierten Oligonukleotide eine PolyT-Sequenz aufweist, die in der Lage ist, an einen PolyA-Schwanz eines ersten Messenger-Ribonukleinsäure-(mRNA)-Targets zu binden;
(b) nach einer Doppelstrangsynthese zulassen, dass ein Teil der cDNA einer PCR-Amplifikation und einer Zyklisierung unterzogen wird, um zirkuläre doppelsträngige cDNA zu bilden;
(c) Verwenden der zirkulären doppelsträngigen cDNA als Matrize, um eine Volllängen-Anreicherung eines Zielgens mittels inverser PCR unter Verwendung eines strangspezifischen Primers durchzuführen, der zum Anreichern des Zielgens ausgelegt ist; und
(d) Abgleichen und Analysieren von Transkriptominformationen und Zielgeninformationen.

2. Verfahren nach Anspruch 1, wobei die Sequenz der mehreren barcodierten Oligonukleotide Bindungsstellen für zwei verschiedene Sequenzierprimer aufweist, wobei die Bindungsstellen als PCR-Primerbindungssequenzen zum Amplifizieren der Sequenz der cDNA verwendet werden können.

3. Verfahren nach Anspruch 1, wobei die Sequenz der mehreren barcodierten Oligonukleotide Bindungsstellen für zwei verschiedene Sequenzierprimer aufweist, wobei die Bindungsstellen als Sequenzierprimerbindungsstellen zum Durchführen einer Bibliothekssequenzierung verwendet werden können.

4. Verfahren nach Anspruch 1, wobei die cDNA-Zyklisierung durch eine enzymatische Reaktion durchgeführt wird.

5. Verfahren nach Anspruch 4, wobei das Enzym für die cDNA-Zyklisierung eine DNA-Ligase ist.

6. Verfahren nach Anspruch 4, wobei das Enzym für die cDNA-Zyklisierung eine DNA-Polymerase ist.

7. Verfahren nach Anspruch 1, wobei die Informationen durch Sequenzierung erhalten werden.

8. Verfahren nach Anspruch 1, wobei das Abgleichen und Analysieren durch Paarung nach Identifizierung des Zellbarcodes durchgeführt wird.

9. Verfahren nach Anspruch 1, wobei das Zielgen ein Gen eines Wirbeltiers ist.

10. Verfahren nach Anspruch 1, wobei der strangspezifische Primer zum Anreichern des Zielgens ausgelegt ist, das vom 3'-Ende entfernt ist.

11. Verfahren nach Anspruch 1, wobei die Anreicherung mindestens drei Anreicherungsrunden aufweist.

12. Verfahren nach Anspruch 1, wobei das Zielgen für einen T-Zell-Rezeptor (TCR) oder einen B-Zell-Rezeptor (BCR) codiert.

## Revendications

1. Procédé d'analyse d'un niveau d'expression de gène cellulaire et d'une séquence de gène cible au niveau d'une cellule unique, le procédé comprenant :
(a) le chargement d'une cellule et d'une bille fixée à des multiples oligonucléotides à code-barres ensemble dans un même micropuits, dans lequel chacun des multiples oligonucléotides à code-barres comprend un code-barres de cellule et un identificateur moléculaire unique (UMI), dans lequel chacun des premiers oligonucléotides à code-barres des multiples oligonucléotides à code-barres comprend une séquence polyT capable de se lier à une queue polyA d'une première cible d'acide ribonucléique messager (ARNm) ;
(b) après une synthèse double brin, le fait de permettre à une partie de l'ADNc de subir une amplification et une cyclisation par PCR pour former un ADNc double brin circulaire ;
(c) l'utilisation de l'ADNc double brin circulaire comme modèle pour effectuer un enrichissement de pleine longueur d'un gène cible par PCR inverse à l'aide d'une amorce spécifique au brin conçue pour enrichir le gène cible ; et
(d) la mise en correspondance et l'analyse d'informations de transcriptome et d'informations de gène cible.

2. Procédé selon la revendication 1, dans lequel la séquence des multiples oligonucléotides à code-barres comprend des sites de liaison pour deux amorces de séquençage différentes, dans lequel les sites de liaison peuvent être utilisés comme séquences de liaison d'amorce PCR pour amplifier la séquence de l'ADNc.

3. Procédé selon la revendication 1, dans lequel la séquence des multiples oligonucléotides à code-barres comprend des sites de liaison pour deux amorces de séquençage différentes, dans lequel les sites de liaison peuvent être utilisés comme sites de liaison d'amorce de séquençage pour effectuer un séquençage de bibliothèque.

4. Procédé selon la revendication 1, dans lequel la cyclisation d'ADNc est effectuée par une réaction enzymatique.

5. Procédé selon la revendication 4, dans lequel l'enzyme pour la cyclisation d'ADNc est une ADN ligase.

6. Procédé selon la revendication 4, dans lequel l'enzyme pour la cyclisation d'ADNc est une ADN polymérase.

7. Procédé selon la revendication 1, dans lequel les informations sont obtenues par séquençage.

8. Procédé selon la revendication 1, dans lequel la mise en correspondance et l'analyse sont effectuées par appariement après identification de code-barres de cellule.

9. Procédé selon la revendication 1, dans lequel le gène cible est un gène d'un vertébré.

10. Procédé selon la revendication 1, dans lequel l'amorce spécifique au brin est conçue pour enrichir le gène cible éloigné de l'extrémité 3'.

11. Procédé selon la revendication 1, dans lequel l'enrichissement comprend au moins trois cycles d'enrichissement.

12. Procédé selon la revendication 1, dans lequel le gène cible code pour le récepteur de lymphocytes T (TCR) ou le récepteur de lymphocytes B (BCR).
